(19) Europäisches Patentamt
European Patent Office
Office européen des brevets

(11) EP 0 656 893 B1

(12) **EUROPÄISCHE PATENTSCHRIFT**

(45) Veröffentlichungstag und Bekanntmachung des Hinweises auf die Patenterteilung:
**12.12.2001 Patentblatt 2001/50**

(21) Anmeldenummer: **94918748.8**

(22) Anmeldetag: **28.06.1994**

(51) Int Cl.[7]: **C07D 265/34**, C07D 265/36, C07D 265/12, C07D 498/04, G03C 1/685
// (C07D498/04, 265:00, 221:00)

(86) Internationale Anmeldenummer:
**PCT/DE94/00744**

(87) Internationale Veröffentlichungsnummer:
**WO 95/00500 (05.01.1995 Gazette 1995/02)**

(54) **PHOTOCHROME OXAZINEVERBINDUNGEN**

PHOTOCHROMIC OXAZINE COMPOUNDS

COMPOSES PHOTOCHROMES D'OXAZINE

(84) Benannte Vertragsstaaten:
**DE ES FR GB IT**

(30) Priorität: **28.06.1993 DE 4321461**

(43) Veröffentlichungstag der Anmeldung:
**14.06.1995 Patentblatt 1995/24**

(73) Patentinhaber: **Optische Werke G. Rodenstock
80469 München (DE)**

(72) Erfinder:
- **MELZIG, Manfred
D-82234 Wessling (DE)**
- **ZINNER, Herbert
D-93080 Pentling (DE)**

(74) Vertreter: **Münich, Wilhelm, Dr.
Dr. Münich & Kollegen
Anwaltskanzlei
Wilhelm-Mayr-Strasse 11
80689 München (DE)**

(56) Entgegenhaltungen:
**EP-A- 0 449 669**
**WO-A-87/00524**
**WO-A-92/09593**
**WO-A-93/10112**
**WO-A-93/17071**

- **CHEMICAL ABSTRACTS, vol. 120, no. 20, 16. Mai 1994, Columbus, Ohio, US; abstract no. 257519p, NAKAMURA M. ET AL 'Polymerizable photochromic substances and polymeric photo chromic materials.' & JP,A,0 598 252 (TORAY INDUSTRIES) 20. April 1993**
- **CHEMICAL ABSTRACTS, vol. 98, no. 16, 18. April 1983, Columbus, Ohio, US; abstract no. 135279s, REICHENBAECHER M. ET AL 'Photochromic media containing 1,4-2H-oxazines and spiro phenanthro-1,4-2H-oxazines' & DD,A,156 372 18. August 1982**
- **CHEMICAL ABSTRACTS, vol. 97, no. 20, 15. November 1982, Columbus, Ohio, US; abstract no. 164536h, REICHENBAECHER M. ET AL 'Photochromic 1,4-2H-oxazines and spirophenanthro-1,4-2H-oxazines' & DD,A,153 690 27. Januar 1982**
- **TETRAHEDRON LETTERS, Bd.22, Nr.40, 1981, OXFORD GB Seiten 3945 - 3948 U.-W. GRUMMT 'New photochromic 2H-1,4-oxazines and spiro-2H-1,4-oxazines.'**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 656 893 B1

## Beschreibung

Die Erfindung betrifft photochrome Oxazinverbindungen sowie ein Verfahren zur Herstellung derartiger Oxazinverbindungen.

Anwendungszweck dieser Verbindungen ist insbesondere das Einfärben von optischen Elementen aus einem Kunststoffmaterial.

Pyrane sind eine seit langem bekannte und gut untersuchte Klasse von photochromen Farbstoffen. So haben seit kurzem entwickelte photochrome Farbstoffe, wie die in der US-A-5 066 818, der WO 92/09593 oder der US-A-4 818 086 beschriebenen Diphenylnaphthopyrane in einem weiten Rahmen einstellbare Kinetikeigenschaften. Ihre Lebensdauer, d.h. ihre Lichtstabilität im Dauergebrauch ist jedoch für viele Anwendungen ungenügend.

Spiropyrane, die in 2-Stellung spiroverknüpft sind, zeigen ebenfalls ungenügende Lichtstabilität. Dies gilt sowohl für die in der US-A-4 818 096 beschriebenen Adamantanderivate wie auch für die in der US-A-5 106 998 beschriebenen Derivate vornehmlich tricyclischer Kohlenwasserstoffe.

Die wegen ihrer ausgezeichneten Stabilität bislang bevorzugten Verbindungen aus der Klasse der Indolino-(spiro-benzoxazine oder -naphthoxazine), wie sie z.B. in der US-A-3 652 172, der US-A-3 578 602, der US-A-4 215 010 oder der US-A-4 637 698 beschrieben sind, sind hingegen bezüglich Eindunkelungs- und Aufhellgeschwindigkeit nur geringfügig beeinflußbar.

In Chemical Abstracts 97:164536h (1982) ist die Herstellung eines photochromen 1,4-2H-Oxazins der Formel

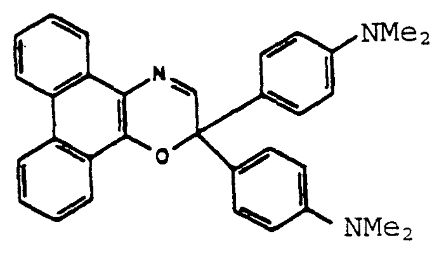

durch die Kondensation von Phenanthrenchinonmonooxim oder Naphthochinonmonooxim oder deren Derivate mit 1,1-bis(p-Dialkylaminophenyl)ethylen, 1,1-bis(p-Alkoxyphenyl)ethylen, alkylidensubstituierten Benzoheterocyclen oder Oniumvorläufern der genannten Benzoheterocyclen in Gegenwart von sauren oder basischen Katalysatoren beschrieben.

In Tetrahedron Letters 22, Nr. 40, S. 3945 bis 3948 (1981) werden photochrome 2H-1,4-Oxazine der nachstehenden Formeln

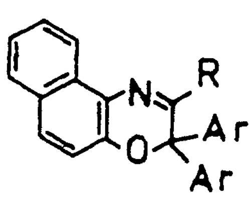

und

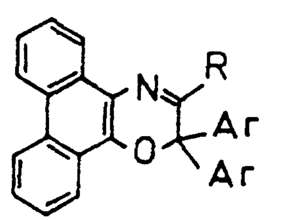

beschrieben, in denen

Ar = 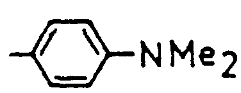

und R = H oder Me.

Zur Synthese dieser Verbindungen wird 1-Nitroso-2-naphthol oder Phenanthrenchinon-monoxim mit 1.1-bis [p-(Dimethylamino)phenyl]ethylen bzw. 1,1-[p-(Dimethylamino)phenyl]-2-methyl-ethylen in absolutem Ethanol in Gegenwart katalytischer Mengen Eisessig unter Rückfluß umgesetzt.

Der Erfindung liegt die Aufgabe zugrunde, einen alternativen Syntheseweg für photochrome Oxazinverbindungen anzugeben, die die vorteilhaften Eigenschaften von Pyranen und insbesondere von Diphenylnaphthopyranen bei einer Lebensdauer bzw. Lichtstabilität aufweisen, die mit der hohen Lebensdauer, d.h. Lichtstabilität der - vornehmlich von Indolinospironaphtho- bzw. -benzderivaten her bekannten - Oxazine vergleichbar ist.

Die erfindungsgemäße Lösung dieser Aufgabe ergibt sich aus den Patentansprüchen.

Erfindungsgemäß wird die Aufgabe durch Kondensation von substituierten Diphenylacetaldehyden (Reaktionspartner 1) mit (substituierten) ortho-Aminohydroxyaromaten (Reaktionspartner 2) gelöst. Hierdurch erhält man eine Substanzklasse, die die geforderten positiven Eigenschaften aufweist. Erforderlich ist eine in $\alpha$-Stellung zur Aldehydgruppe befindliche Halogen- oder Hydroxygruppe, die mit der Hydroxygruppe des Reaktionspartners 2 unter Austritt von Wasser oder Halogenwasserstoff kondensiert.

Selbstverständlich können auch einer oder beide Phenylreste weiter substituiert sein.

Die Reaktion ist auch mit (substituierten) Dinaphthyloder Phenylnaphthylderivaten durchzuführen; auch Heteroaromaten sind verwendbar.

Die beiden Phenylringe können auch chemisch starr verknüpft sein (z.B. Fluoren-, Dibenzosuberon-, Anthron-, Xanthen- oder Thioxanthenderivate), wie dies in einer am gleichen Tag eingereichten Anmeldung desselben Anmelders beschrieben ist.

Die kinetischen Eigenschaften der erfindungsgemäß hergestellten Verbindungen lassen sich in analoger Weise zur Vorgehensweise gemäß WO 92/09593 vor allem durch Substituenten in 2-Stellung der aromatischen Ringe beeinflussen.

Der zweite Reaktionspartner besitzt besonders bevorzugt eine ortho-Aminohydroxy-Gruppierung an einem aromatischen Ringsystem. Dieses kann ein evtl. weiter substituiertes Benzol, Naphthalin, Phenanthren, Anthracen oder beispielsweise Chinolin oder Isochinolin aus der Reihe der Heteroaromaten sein.

Bevorzugt sind 1-Amino-2-naphthole, wobei sich durch geeignete Substitution in 3-Stellung, analog zur US-A-5,066,818, die kinetischen Eigenschaften leicht beeinflussen lassen.

Die Kondensation selbst kann mit der freien Base, dem Hydrochlorid oder unter Abwandlung der Vorschrift auch mit einer beispielsweise durch eine Acetylgruppe geschützten Aminogruppen durchgeführt werden.

Als Lösemittel sind insbesondere solche geeignet, die mit Wasser ein niedriger siedendes Azeotrop bilden, so daß sich das Reaktionswasser beispielsweise mittels einer Dean-Stark-Apparatur leicht entfernen läßt. Der Halogenwasserstoff wird ausgetrieben oder beispielsweise in einem Extraktor durch festes Alkalihydroxid gebunden. Je nach eingesetzten Reaktionspartnern wird die Reaktion vorzugsweise basisch oder sauer (p-Toluolsulfonsäure) katalysiert.

**Beschreibung bevorzugter Ausführungsbeispiele**

Syntheseweg

Im nachstehenden wird eine Synthese beschrieben, die vom 1-Amino-2-naphthol ausgeht. Alle Verbindungen der Tabelle 1 wurden in analoger Weise synthetisiert. Die entsprechenden Nitrosoderivate können auch durch Reduktion der Nitro-derivate erhalten werden (vgl.Liebermann und Jacobson, Ann.211, 48 (1882)). Dieser Weg empfiehlt sich wegen der vornehmlich entstehenden 4-Nitro-1-naphthole in der Vorstufe für die Verbindungen 1-3 nicht. Statt der Halogen-Acetaldehydverbindungen können im Prinzip auch die analogen Hydroxyverbindungen eingesetzt werden, die Ausbeuten sind jedoch i.a. deutlich geringer.

Die für die Verbindungen 3,7 und 9 benötigten methylsubstituierten Diphenyl- bzw. Naphthylphenylacetaldehyde sind nach Literaturvorschriften darstellbar.

1. Bromierung von Diphenylacetaldehyd:

25 g (0.13 mol) Diphenylacetaldehyd werden unter Stickstoffspülung in 100 ml von Sauerstoff befreitem Tetrachlorkohlenstoff gelöst. Die Lösung wird auf -5° C gekühlt und langsam 21 g (0.26 mol) Brom zugetropft. Die Temperatur soll -3° C nie überschreiten. Die Lösung färbt sich braun, wobei sich die anfangs schnelle Bromentfärbung mit der Zeit deutlich verlangsamt. Unter Erwärmung auf etwa 5° C wird das nicht umgesetzte Brom mit Stickstoff ausgetrieben, bis eine honiggelbe Lösung verbleibt. Nach dem Abzug des $CCl_4$ erhält man 30,8 g eines gelben Öles.

2. Reduktion von Nitrosonaphthol:

48 g 1-Nitroso-2-naphthol werden in einer Mischung von 300 ml Wasser und 60 ml 5n NaOH suspendiert, dann werden weitere 240 ml 5n NaOH zugesetzt. Man leitet unter gutem Rühren heißen Wasserdampf durch die Lösung, bis die Temperatur 35° C erreicht hat. Dann werden 120 g frisch zubereitetes Natriumdithionit zugegeben,

wobei die Temperatur schnell auf 60° C steigt. Die Lösung nimmt eine bernsteinfarbene Färbung an. Nach 15 min kühlt man durch Zugabe von 200 g Eis schnell auf unter 20° C ab und gibt 100 ml konz. Salzsäure zu. Der weiße, flockige Niederschlag wird abgenutscht und in einer Mischung von 500 ml Wasser und 50 ml konz.Salzsäure suspendiert. In diese Mischung wird überhitzter Wasserdampf mit solch einem Volumenstrom eingeleitet, daß die Temperatur binnen 10 min 90° C erreicht. Bei dieser Temperatur wird unter Reduzierung des Dampfstromes noch 1 h gerührt. Die noch heiße Lösung wird filtriert, wobei etwas auberginenfarbener Niederschlag zurückbleibt. Beim Abkühlen des Filtrats fällt ein heller, flockiger Niederschlag aus, der abgesaugt, mit verdünnter Salzsäure und danach mit Ether gewaschen und getrocknet wird. Es wurden 45 g 1-Amino-2-naphthol-hydrochlorid erhalten. Die Aminohydroxyaromaten wurden bei allen Umsetzungen in dieser sauren Form verwendet, da sich die freien Basen - mit Ausnahme des 5-Amino-6-chinolinols - als sehr instabil erwiesen.

3. Darstellung der Oxazinverbindung:

10 g (51 mmol) 1-Amino-2-naphthol-hydrochlorid werden mit 14 g (53 mmol) Diphenyl-bromacetaldehyd in 200 ml Toluol unter Zusatz einer Spatelspitze p-Toluolsulfonsäure und unter Rühren am Wasserabscheider gekocht. Wenn kein weiteres Reaktionswasser mehr gebildet wird, läßt man die Lösung abkühlen. Nach dem Abzug des Lösemittels wird das Rohprodukt mit Methylenchlorid an Aluminiumoxid chromatographiert.

Die rot laufende Fraktion enthält die photochrome Verbindung, die auf Filterpapier unter UV-Licht eine intensiv rotviolette photochrome Färbung zeigt. Nach dem Abziehen des Laufmittels wird das Produkt aus Diethylether / Hexan umkristallisiert. Man erhält 5,4 g eines rotstichigen Pulvers, das durch NMR-Analyse als 2,2-Diphenyl-2H-naphth(2,1-b) oxazin identifiziert wird (Beispiel 4).

Herstellung der Meßproben

Zum Vergleich sind zu einigen in Tabelle 1 genannten Verbindungen die analogen Verbindungen aus dem Stand der Technik synthetisiert worden. Diese unterscheiden sich in der Struktur durch den Austausch des Oxazin-Stickstoffatoms gegen einen CH-Rest, stellen also die zu den Oxazinen analogen Pyrane dar.

Mit den erfindungsgemäß hergestellten Verbindungen sowie den Vergleichssubstanzen werden im Handel erhältliche Nullgläser aus Polydiethylengkykolbisallylcarbonat (Durchmesser 71 mm, Mittendicke ca. 2 mm) mit dem in der DE 35 16 568 A1 beschriebenen Verfahren konvexseitig eingefärbt.

Meßergebnisse

Die wie vorstehend beschrieben hergestellten Gläser sind in einer Meßbank entsprechend DIN 58 217 vermessen worden. Kennwert ist dabei das spektrale Helligkeitsempfinden des Normalbeobachters. Die nach $V_\lambda$ bewertete Differenz zwischen den Transmissionen $\tau_0$ im ausgebleichten Zustand und den Transmissionen $\tau_s$ im angeregten Zustand nach 15 min Belichtung bei 23°C in einer temperierten Küvette stellt in der Form $\Delta$OD den Ausgangswert dar:

$$\Delta OD = 10 \log \tau_0 - 10 \log \tau_s.$$

Nach einer Belichtung von 100 h im Bestrahlungstestgerät (Suntest, Original Hanau) mit etwa 100 klux und sonnenähnlicher Spektralverteilung wird diese Differenz erneut ermittelt.

Der Quotient

$$R = (\Delta OD\ 100\ h) : (\Delta OD\ 0\ h)$$

ist die verbliebene Restleistung. Sie ist auf den Anfangswert bezogen und stellt ein direktes Vergleichsmaß für die Bestrahlungsbeständigkeit dar, da alle Unterschiede bzgl. spektraler Anregung und Absorption, Färbevermögen des Kunststoffmaterials etc. keine Auswirkung haben. Die Ergebnisse sind in Tabelle 2 dargestellt.

Tabelle 1 :

| Substitutionsschema der erfindungsgemäß hergestellten photochromen Verbindungen | | | | | | |
|---|---|---|---|---|---|---|
| Beispiel | Formel | R1 | R2 | R3 | R4 | R5 |
| 1 | A | H | H | H | H | H |

Tabelle 1 : (fortgesetzt)

| Substitutionsschema der erfindungsgemäß hergestellten photochromen Verbindungen | | | | | | |
|---|---|---|---|---|---|---|
| Beispiel | Formel | R1 | R2 | R3 | R4 | R5 |
| 2 | A | H | H | H | H | $OCH_3$ |
| 3 | A | H | 2-$CH_3$ | H | H | H |
| 4 | B | H | H | H | H | H |
| 5 | B | H | H | H | H | $OCH_3$ |
| 6 | B | H | 2-$CH_3$ | H | H | H |
| 7 | B | H | H | $OCOCH_3$ | H | H |
| 8 | B | OCH3 | 2,3-benzo | H | H | H |
| 9 | C | H | H | H | H | H |
| 10 | C | H | H | H | $C_6H_5$ | H |
| 11 | D | H | H | H | H | H |
| 12 | E | H | H | H | H | H |

Im folgenden sind die Strukturformeln A bis E dargestellt, an denen sich die in der Tabelle 1 aufgeführten Reste befinden:

A:

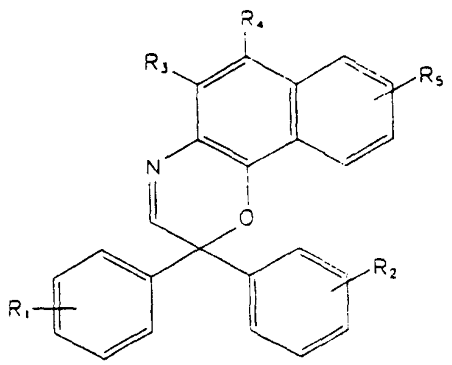

B:

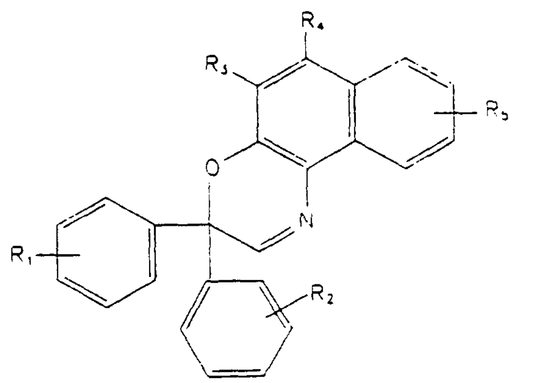

C:

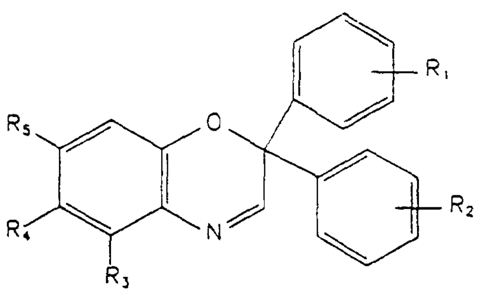

D:

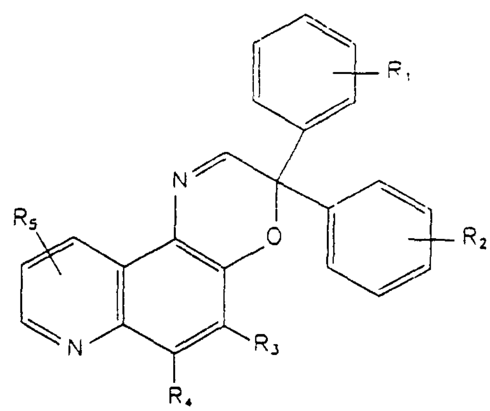

E:

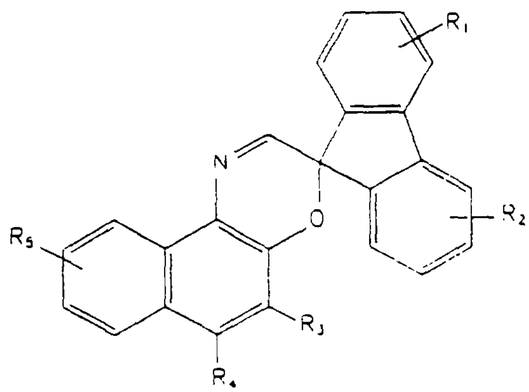

Tabelle 2 : Lebensdauern der einzelnen Verbindungen

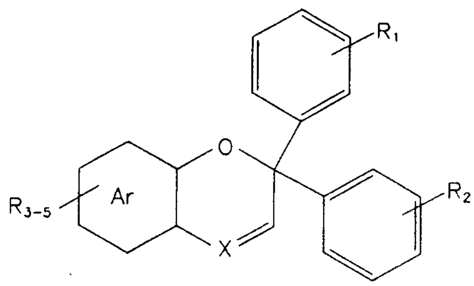

| Erfindungs-beispiele (X = N) | R ( % ) | Vergleichs-beispiele (X = CH) | R ( % ) |
|---|---|---|---|
| 1 | 70 | 1' (US-A-3 567 605) | 54 |
| 4 | 78 | 4' (US-A-3 627 690) | 56 |
| 5 | 62 | 5' (WO 93/17071) | 45 |
| 6 | 62 | 6' (WO 92/09593) | 41 |
| 7 | 69 | 7' (US-A-5 066 818) | 47 |

**Patentansprüche**

**1.** Verfahren zur Herstellung photochromer Oxazinverbindungen, bei dem ein Diphenyl-, Phenylnaphthyl- oder Dinaphthylacetaldehyd oder ein mit Heteroaromaten substituierter Acetaldehyd, der in α-Stellung zur Aldehydgruppe eine Halogen- oder Hydroxygruppe trägt und substituiert sein kann, mit einem ortho-Aminohydroxyaromaten, der substituiert sein kann, kondensiert wird.

2. Verfahren nach Anspruch 1, bei dem das Kohlenstoffatom, welches die Aldehyd- und die Halogen- oder Hydroxygruppe trägt, ein Teil eines Ringsystems ist.

3. Verfahren nach Anspruch 2, bei dem das Ringsystem Fluoren, Dibenzosuberon, Xanthen oder Thioxanthen ist.

4. Verfahren nach Anspruch 2, bei dem das Ringsystem ein aliphatischer, eingliedriger Ali- oder Heterocyclus ist.

5. Verfahren nach Anspruch 4, bei dem das Ringsystem Cyclohexan oder Tetrahydropyran ist.

6. Verfahren nach Anspruch 2, bei dem das Ringsystem ein polycyclischer Kohlenwasserstoff ist.

7. Verfahren nach einem der Ansprüche 1 bis 6, bei dem der ortho-Aminohydroxyaromat ein 1-Amino-2-naphthol oder 2-Amino-1-naphthol ist, das substituiert sein kann.

8. Verfahren nach einem der Ansprüche 1 bis 6, bei dem der ortho-Aminohydroxyaromat 5-Amino-6-hydroxychinolin oder 6-Amino-5-hydroxychinolin ist.

9. Verfahren nach einem der Ansprüche 1 bis 6, bei dem der ortho-Aminohydroxyaromat 5-Amino-6-hydroxyisochinolin oder 6-Amino-5-hydroxyisochinolin ist.

10. Verfahren nach einem der Ansprüche 1 bis 6, bei dem der ortho-Aminohydroxyaromat eine ortho-Aminohydroxygruppierung an einem aromatischen Ringsystem besitzt, das Benzol, Naphthalin, Phenanthren, Anthracen, Chinolin oder Isochinolin ist, das weiter substituiert sein kann.

## Claims

1. Method for production of photochromic oxazine compounds, in which a diphenyl-, phenylnaphthyl- or dinaphthylacetaldehyde or an acetaldehyde which is substituted with heteroaromatics, which acetaldehyde carries a halogen- or hydroxy group in -position relative to the aldehyde group and which can be substituted, is condensed with an orthoaminohydroxyaromatic which can be substituted.

2. Method according to claim 1, in which the carbon atom which carries the aldehyde-and the halogen- or hydroxy group is part of a ring system.

3. Method according to claim 2, in which the ring system is fluorene, dibenzosuberone, xanthene or thioxanthene.

4. Method according to claim 2, in which the ring system is an aliphatic, monomial alior heterocycle.

5. Method according to claim 4, in which the ring system is cyclohexane or tetrahydropyrane.

6. Method according to claim 2, in which the ring system is a polycyclic hydrocarbon.

7. Method according to one of the claims 1 to 6, in which the orthoaminohydroxyaromatic is a 1-amino-2-naphthol or 2-amino-1-naphthol which can be substituted.

8. Method according to one of the claims 1 to 6, in which the orthoaminohydroxyaromatic is 5-amino-6-hydroxyquinoline or 6-amino-5-hydroxyquinoline.

9. Method according to one of the claims 1 to 6, in which the orthoaminohydroxyaromatic is 5-amino-6-hydroxyisoquinoline or 6-amino-5-hydroxyisoquinoline.

10. Method according to one of the claims 1 to 6, in which the orthoaminohydroxyaromatic has an orthoaminohydroxy grouping on an aromatic ring system which is benzol, naphthalene, phenanthrene, anthracene, quinoline or isoquinoline which can be further substituted.

**Revendications**

1. Procédé de préparation de composés photochromes d'oxazine, dans lequel un diphényl-, phénylnaphtyl- ou dinapthylacétaldéhyde ou un acétaldéhyde substitué par des hétéroatomes, qui porte un groupe halogène ou hydroxyle dans la position $\alpha$ par rapport au groupe aldéhyde et qui peut être substitué, est condensé avec un composé ortho-aminohydroxyaromatique qui peut être substitué.

2. Procédé selon la revendication 1, dans lequel l'atome de carbone qui porte le groupe aldéhyde et le groupe halogène ou hydroxyle fait partie d'un système cyclique.

3. Procédé selon la revendication 2, dans lequel le système cyclique est le fluorène, la dibenzosubérone, le xanthène ou le thioxanthène.

4. Procédé selon la revendication 2, dans lequel le système cyclique est un composé alicyclique ou hétérocyclique aliphatique à un terme.

5. Procédé selon la revendication 4, dans lequel le système cyclique est le cyclohexane ou le tétrahydropyrane.

6. Procédé selon la revendication 2, dans lequel le système cyclique est un hydrocarbure polycyclique.

7. Procédé selon l'une des revendications 1 à 6, dans lequel le composé ortho-aminohydroxyaromatique est un 1-amino-2-naphtol ou un 2-amino-1-naphtol qui peut être substitué.

8. Procédé selon l'une des revendications 1 à 6, dans lequel le composé ortho-aminohydroxyaromatique est la 5-amino-6-hydroxyquinoléine ou la 6-amino-5-hydroxyquinoléine.

9. Procédé selon l'une des revendications 1 à 6, dans lequel le composé ortho-aminohydroxyaromatique est la 5-amino-6-hydroxyisoquinoléine ou la 6-amino-5-hydroxyisoquinoléine.

10. Procédé selon l'une des revendications 1 à 6, dans lequel le composé ortho-aminohydroxyaromatique possède un groupement ortho-aminohydroxyle sur un système cyclique qui est le benzène, le naphtalène, le phénanthrène, l'anthracène, la quinoléine ou l'isoquinoléine et qui peut être encore substitué.